# EUROPEAN PATENT APPLICATION

(11) **EP 2 311 517 A1**
(43) Date of publication of application: **20.04.2011**
(21) Application number: 10187204.2
(22) Date of filing: 12.10.2010
(51) Int. Cl.: A61M 25/00

(54) **Combined catheter for angiography.**

(30) Priority: 15.10.2009 AR P010003980
(71) Applicant: Bettinotti, Marcelo Omar, 1417 Buenos Aires (AR)
(72) Inventor: Bettinotti, Marcelo Omar, 1417 Buenos Aires (AR)
(74) Representative: Robba, Pierpaolo

(57) **Abstract**

It is a specially conceived catheter to perform a coronary catheterization by the amoral route with a prior punction and placing of 2mm introductors, using the "Judkins'" catheter curves but without the need of first advancing the JL catheter and then the JR. It comprises a "Judkins" JR right coronary catheter, which contains in its interior a "Judkins" JL left coronary catheter that distally protrudes, disposing the section, defining the primary and secondary curves projecting from the distal end of the JR catheter of the right coronary, which is tapered and blunt in its own distal end section. It is emphasized that the JR catheter has incorporated a haemostatic valve hand in hand with its proximal end.

## Description

The present invention has, as a main objective, a combined catheter for angiography, comprising a right coronary (JR) mainly with a 2 mm diameter, with an haemostatic valve, specially designed to contain, in its interior, a left coronary catheter, mainly with a 1.35 mm diameter.

More specifically, the present patent invention comprises a combined catheter that may be defined as "two-in-one catheter" from which it is possible to perform a coronary catheterization by femoral route, with a prior puncture and placing 2mm introducers, using the "Judkins" catheters curves but without the need of first advancing the JL catheter and then the JR catheter.

The main functional advantage rendered by the combined catheter lies in the fact that it allows to advance both catheters at the same time and to carry out the complete test, since a JL 1.35 mm curved catheter is advanced within a JR 2 mm valved catheter, specially designed to contain the latter, which allows us to avoid the exchange of catheters and/or strings.

It is highlighted that, with the use of the combined catheter of the present invention, the user may first catheterize the left coronary, then partially remove the JL and advance the JR catheter and then selectively catheterize the right coronary artery, all of them in a sole act.

With the new invented combined catheter, different projections may be carried out, being able to quickly finish with the coronary angiography.

It is an invention that defines a new media combination conceived to achieve a higher result, being the same unpredictable and surprising, even for a skilled person. Consequently, besides being novel, its constructive and inventive conception shows a clear inventive activity, thus, it meets all the conditions required by Law to be deemed as a patent of invention.

### BACKGROUND ART

### CINECORONAROGRAPHY BY FEMORAL ROUTE

### Femoral artery approach

The approach by the femoral artery is more frequently used. It requires a complete knowledge of the peripheral circulation anatomy and control on the percutaneous vascular catheterism.

A special needle is introduced, with an appropriate blunt fill and an obturator, through the femoral artery. When a good pulsatile flow is observed, a guide is introduced (generally with a diameter of 0.035 inches) through the needle, advancing the same in the arterial system.

After the fluoroscopy examination is made, to assure that the guide has advanced in a retrograde way (i,e., towards the iliac artery and the aorta), pressure is exercised with the third and fourth toes upon the artery proximal to the insertion place and the needle is removed with the right hand; then, an appropriate artery dilator and a sheath with a valve for catheter introduction are introduced. This helps the catheter change and minimizes the artery damage.

Then, the selective coronary catheterization is performed, using the special catheters and the techniques that will be described below.

### Catheters and Judkins technique

These pre-formed, right and left coronary catheters usually are made of polyurethane or polyethylene, with stainless steel metallic meshes or sashes. Generally, the coronary arterial catheters are 100 cm. long and have a diameter of 1.67 to 2.7 mm. They have only one end hole and there are variations in the shape of its distal end. The pre-formedurves have been designed upon the concept of the proximal and transversal portions of the aortic arch, the ascending aorta and the orifice of the left coronary that are located approximately at the same level.

The body of the catheter advancing towards the aortic root from below tends to lie against the aortic wall in the opposite direction of its tip. Like that, the operator has to choose the appropriate size catheter before attempting the procedure. This is made by assessing the body habits of the patient together with the medical findings (e.g., aortic stenosis) and the size and shape of the aortic root, through a thorax x-ray or a fluoroscopy examination.

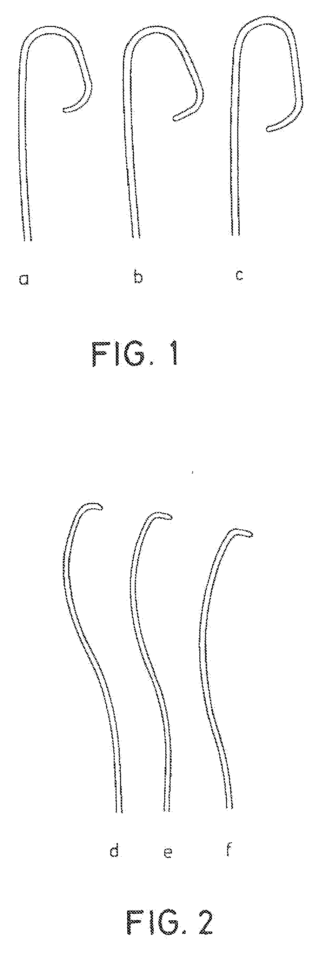

The "Judkins" catheters for the left coronary have primary (90º), secondary (180°) and tertiary (see Fig.1 in Description of a Preferred Example) curves. The radius of the curvature of the latter is of approximately 10 cm., while the distance between the primary and secondary curves varies between 3.5 cm. and 6 cm. The size of the catheter for the left coronary is determined by the distance between the primary and secondary curves and that also determines the angle formed by the distal segment and the coronary artery. This catheter size is applicable to most of the normal aortas and can be used in most of the patients.

The distance between the primary and secondary curves is around 5 cm. in "Judkins" N° 5, while in the N° 6 the distance between the primary and secondary curves is of 6 cm., and it is useful for the dilated, big, poststenotics aortas of the patient with aortic valvular stenosis or disease of the aortic root (e,g., patients with Marfan syndromes or syphilis).

Occasionally, N° 7 catheters are needed and they can be built with a heat gun.

In child or small build adults, particularly young women, N° 3 or 3.5 catheters are used.

Also, the right coronary Judkins catheter has a secondary curve of 135°, with a radius of 5 to 6 cm. for small and medium sizes, and approximately of 11 cm. for big sizes (See Fig. 2 in the Description of a Preferred Example).

Generally, the left Judkins catheter is used first (after the ventriculography). The pressure is controlled when the catheter advances towards the aortic arch. The catheter is advanced, with the patient in a light LAO projection so that the tip slides through the left wall of the ascending aorta until it "falls" in the Valsalva sinus and left coronary orifice. The pressure wave is inspected to search for alterations or "damping of its layout". When absent, a test injection (1 to 2 cc) is observed in the fluoroscope. During this test, the operator has to pay particular attention to the position of the catheter in the left coronary artery.

The angle formed by the tip of the Judkins catheter with the direction of the proximal coronary artery shall be minimum, to assure that it does not touch the artery wall. This is very important in order to avoid dissecting the coronary artery if the catheter moves in an unexpected way to a deeper position within the artery, for example, when the patient exhales. It is essential to inject contrast only in the left coronary sinus before the selective opacification in case any obstruction of the left coronary trunk is suspected (e.g., calcification, unstable angina, electrocardiographic changes marked during the ischemia or decrease of the systolic pressure waves width when selectively catheterizing the trunk).

After performing the multiple injections, with filming in different projections, the left catheter is changed for the right coronary. Generally, this catheter is advanced until its tip is in the upper position of the left sinus. Then, the clockwise rotation ("hourly") is used to move the tip in the anterior direction, until it enters the right orifice. This maneuver is better performed in LAO projection at 20°.

If the operator has any difficulty catheterizing the coronary orifice with the preselected catheter, it is important not to lose time trying to manipulate a catheter of an inappropriate size in the coronary orifice. A test injection has to be performed close to the coronary orifices to evaluate its position and size as well as the size and shape of the desired catheter, replacing the catheter that has been placed for other of different size and shape.

Generally, the selective catheterism of the coronary arteries is easy and quick to perform (in 1 min.), even if it is performed by less skilled operators using the Judkins catheter.

The best advantage of the Judkins technique is its simplicity. It requires less skill and training than other techniques. Since the catheters have only one terminal orifice, the obstruction of the tip produces an alteration of the shape of the pressure wave.

Recognizing the change of the pressure wave shape may avoid the injection or embolization of a clot by the operator, damaging the intimae of the coronary The obstructive disease of the descending, iliac aorta or the femoral vessels may difficult or prevent the catheterism and may constitute a high risk of arterial damage, thrombosis and cholesterol embolism. Multiple catheters shall be used, which increases the possibility of an arterial damage and/or embolism.

If it were necessary to re-examine both coronaries, for example, after nitroglycerin and ergonovine or during thoracic pain, the catheters should be changed.

### DEFECTS AND RISKS:

Complications, risks and prevention:
The practice of the cardiac catheterism and the angiography has continued evolving throughout its 60 years of existence. The current practices are basically different from the ones of the 50's and beginning of the 60's decade, when the main studied diseases where the valvulopathies and the congenital diseases.
Technical advances such as the defibrillation via direct current, closed-chest cardiac massage and temporary pacemakers were introduced. To the end of the 60's decade and the 70's decade great changes were produced in the catheters design, in the electronic, hemodynamic and angiocardiography registry devices and in the knowledge of the cardiovascular physiology.

Also, these changes allowed invasive procedures that were focused in the coronary disease and in the therapeutic interventions via catheterism. At the beginning, the truly sick or unstable patients were not studied.

Now, given the mentioned advances, neither the acute myocardial infarction nor the unstable angina are contraindications for the study. The possibility of recovering the ischemic myocardium has redefined the indications and the practice of the invasive cardiology.

As a result of this evolution, the complications associated to the cardiac catheterism and the angiography have changed. They reflect the technological skill and advances achieved through the years as well as the challenge of treating patients that have more complex problems and are under a higher risk.

Main studies regarding the catheterism-related complications:
General procedure of the catheterism:
   Several retrospective works that have studied the impact of complications during the catheterism procedures in general, in which the catheterism of the left cardiac cavities and the left ventriculography were included, have been published.

Nevertheless, the experience has proved that the prospective studies provide much more precise information, covering a wide range of pathologies in groups and subgroups representatives of the patients. Four studies agree with these criteria and show a clear image of the contemporary catheterism practice. They are: a) report on complications of the coronary arteriography of the "Coronary Artery" "Surgery Study (CASS)", of 1979, b) report on the coronary arteriography and revascularization surgery of CASS, of 1983, c) records of reports of the "Society for Cardiac Angiography (SFCA)", of 1982 and d) records of data of the SFCA gathered from July 1984 until June 1987, that have not been previously informed.

The CASS study enlisted approximately 25000 patients, from 1974 until 1979. The report included 7533 consecutive patients, to whom a coronary catheterization was performed in 15 institutions. The report of 1983 included data of 19309 patients, to whom a coronary catheterization was performed. The report of 1982 from the Registry Committee of the SFCA was based in 53581 studies performed in 66 laboratories, in a period of 14 months, ending in 1981, who reported their experience after entering in the registry in several locations. The report of the SFCA of 1987 includes data from 199176 patients, 156853 of whom were subjected to coronary angiographic procedures, informed by 70 laboratories according to the protocol established by the Registry Committee of the SFCA. These prospective studies represent the higher number of patients reported until now. They are not identical either in the design or in the composition, but the created subgroups are so great that valid comparisons can be made.

Analysis of the complications:
In the CASS patients, whom only had coronary disease, the total percentage of complications was 1.76% in the report of 1979 and 1.67% in the one of 1983. The studies of the SFCA included all the diagnosis and indications for the catheterism and all possible complications were considered. Nevertheless, the complication percentage was a little higher: 1.82% in the report of 1982 and 1.70% in the one of 1987.

These values are remarkably similar and low, in comparison with the previously published reports, However, they state, in a precise way, the incidence of complications in large series and they may represent acceptable reference standards.

Mortality:
Prior studies, which comprised from 50 up to more than 46000 patients, have reported mortality percentages that vary between 0.03 and 2.6%. The mortality percentages of the CASS and the SFCA were between 0.07 and 0.14% and reflect a great sample of patients and a great variety of laboratory conditions. The risk factors associated with death include disease of the left coronary trunk and multiple vessels, recent unstable angina, a high class of the classification of the "New York Heart Association (NYHA)" or the "Canadian Heart Association", low ejection fraction, recent myocardial infarction, complex congenital malformations, cardiogenic shock, critical aortic stenosis, acute disease of the mitral valve, acute ventricular arrhythmia, high blood pressure and age exceeding the 65 years old.

Deaths for technical errors and for inexperience have become more infrequent. Frequently, the mortality related to the cardiac catheterism is produced in patients with acute cardiovascular disease, commonly, in an advance stage.

Cerebrovascular accident (stroke):
The strokes have a wide variation in its presentation, etiology and duration, according to its localization and size within the affected area. Its occurrence percentage, compared to the cardiac catheterism is of 0.03 to 0.13%.

Even though atheromatous or thrombotic particles give origin to probably, most of the emboli, also the air and foreign bodies have been implicated for detachments of catheter and guide parts during the catheterism. The administration of contrast substance may produce, by itself, seizures, neurological deficit or temporary blindness, even without excessive doses. Fortunately, most of the studies inform that the incidence of the cerebrovascular accidents is lower than the other complications (Table 3-2). The subjects with signs and symptoms of cerebrovascular disease and those with high blood pressure are high risk patients. The treatment for the brain embolism generally includes heparinization.

When there is an air embolism, the hyperbaric oxygen therapy has been successfully used. The control of the hypertension is important.

Vascular complications:
Most of the vascular complications are produced in the place of the catheter insertion or near it, but they can also be the result of embolism remains generated by the catheter or by the manipulation of the guide. These complications are responsible for the 0.44 to 0.83% of the cases.

The vascular occlusions far from the entrance site are infrequent, compared to those that result from the humeral or femoral entry.

It is more likely that the percutaneous puncture of the femoral artery will produce bleeding or haematoma, which require a surgical correction in the operating room with general anaesthesia.

With any technique arteriovenous fistulas, pseudo aneurysms, local nerves injury, infection and pain may be produced.

Other risk is the "Blue Digit Syndrome" or Syndrome of the blue toe, which is characterized by the sudden appearance of pain and purple discoloration of the toes with the presence of palpable peripheral pulse (1). The cyanotic areas may affect the inferior part of the legs, the toes of the feet or of the hands and are clearly outlined by the adjacent skin normally perfused (2). The pathologic alterations are attributed to atheromas and/or embolism of fibrin-platelet aggregation (3-5). In the past, "Flory and Hoye et al" (7) appreciated the embolism events towards the visceral organs and towards the peripheral vessels as derivates of the ulcerating atherosclerotic plaques. Recently, the blue toe syndrome has been discussed by "Karmody et al.", and they have expressed similarities between the clinic manifestations of the blue toe syndrome and the cerebral transitory ischemic attacks (8).

These episodes may be qualified as ischemic episodes of the foot or the hand. In the medical literature (surgical) it has been emphasized that in more than 50 % of the cases, recurrences may appear and they may result in an amputation (5-8). Therefore, the blue toe syndrome shall be treated as a situation where an extremity shall be saved, as in other situations such as rest pain or gangrene (8).

The atherosclerosis injuries to the aorta, iliac artery or femoral vessels may result in macro or microembolizations that may detach due to the advance of the catheters or guides or any other interventional procedure. The mural thrombus may be a source for macroembolisms and may produce acute symptoms. The surgically recovered thrombus may be white or white-pink depending on the organization level.

Histologically, the white thrombus is composed by the different amount of platelets and fibrous material (9). This clinical situation demands a quick and aggressive treatment. The smallest microembolism may represent cholesterol plaques (5) as well as fibrin-platelets wastes of ulcerating atheromatous plaques (8).

These emboli that detach from a superior injury may occlude small vessels in the hand and the foot. An extended and detailed angiography is needed to show said injuries. Nevertheless, the angiographies are rarely practiced in patients with blue toe syndrome with intact pulses and well perfused extremities.

The cholesterol systemic embolization is presented in many different ways and has been called "the great masquerader" (2-10). The atheromatous plaque in the aorta may erode through the intimae, ulcerate and unload cholesterol embolus in the flow towards the peripheral circulation. It may also cause renal insufficiency, high blood pressure, gastrointestinal bleeding, diarrhea, temporary ischemic attacks and myocardial infarction.

The cholesterol embolus in the leg may cause micro-infarctions on the skin, know as livedo reticularis. In patients with digital ischemia for cholesterol embolus, the peripheral pulses are intact. When the peripheral circulation is affected, it may imitate a diffused vasculitis or "hypersensitivity angeitis".

The cholesterol micro-embolizations to the inferior members may cause pain at night, muscular cramps and tingling (12). The biopsy shows occlusion of the arterioles with clefts in the shape of needles containing cholesterol crystals (5, 7, 12). Secondary to the chronic inflammation, there is lymphocyte infiltration, slimming of the media and the intimae, and eventual fibrosis that leads to the last phase of the fibrosis (7,12). Only on the bases of the clinic symptomatology, it is difficult to differentiate between the cholesterol embolism and fibrin-platelets aggregates as the cause of the blue toe syndrome. The cholesterol systemic embolization is easily differentiable of the unilateral syndrome of the blue toe on clinical bases.

Etiological factors:
The blue toe syndrome may be observed in the following pathological states:
   1. In the upper extremity, the source of the embolism is the aneurism of the subclavian artery, "bypass" grafts and radial and lunar (sic) arteries. The secondary thrombus mural under compression of the first anomalous or cervical rib may generate the embolus (thoracic outlet syndrome). The atherosclerotic plaques of stenotic injuries of the subclavian arteries may also embolize towards the digital arteries (13).
   2. Diffused ulcerating injuries of the abdominal aorta and iliac vessels with or without aneurisms.
   3. The stenotic and occlusive injuries of the femoral popliteal system may also shed microembolisms in the digital vessels contributing to the blue toe syndrome. (1, 2, 4, 5, 8, 14-18).
   4. In rare occasions, the thrombotic material in false aneurisms of the thoracic aorta may also shed embolisms contributing to the signs of the blue toes syndrome (19).
   5. The non atherosclerotic injuries such as fibromuscular dysplasia of the external iliac arteries may also generate embolisms (20).

Other complications:
The variety of other possible complications in relation to the cardiac catheterism is huge. Among them we can find acute pulmonary edema, hypotension, shock, vascular or cardiac perforation, cardiac tamponade, endocarditis, internal bleeding, allergic, anaphylactic or by pyrogens reactions, error in drug doses, loss of a member, coronary spasm, aortic aneurism dissecans, renal failure and pulmonary infarction.

All together, these events are infrequent and are rarely individually tabulated. However, they must be considered as part of any report related to catheterism complications. The analysis performed by the CASS specifically excludes the consideration of these complications, meanwhile the registry of the SFCA specifically considers its inclusion. Thus, the global percentage of complications in both reports of the SFCA is quite higher, and maybe more exact, than what has been reported in the CASS.

### SUMMARY OF INVENTION

The combined catheter for angiography to which the present patent applications makes reference, basically consists of a right coronary catheter (JR), with a preferred dimension of a diameter of 2 mm., with a haemostatic valve incorporated (which allows the washing) which has been structurally modified to contain the left coronary catheter JL (of 1.35 mm.) that is currently available in the market, with the special feature that for its incorporation in the femoral artery, the left coronary catheter JL is arranged with its primary and secondary curve sections projected outward from the distal end of the right coronary catheter JR.

The above defined set renders the possibility of performing a coronary catheterization through a femoral way with a previous punction and placing 2 mm. introductors using the Judkins catheter curves but without the need of first advancing the JL catheter and then the JR.

Then, it is emphasized that the main advantage provided by the combined catheter of the present invention is that it allows to advance only once two catheters and performing only one test, since we advance a JL curved catheter of 1.35 mm. inside a modified JR catheter of 2 mm. (valved) and this allows us to avoid the exchange of catheters and/or strings.

It is clear that with the combined catheter of the present invention, it is performed, only in one act, first the catheterization of the left coronary, recorded in several projections and then the partial withdrawn of the JL, the advance of the JR catheter and the selective catheterization of the right coronary artery; therefore, in the same way different projections are made, being able to quickly finish with the coronary angiography.

In this way, we avoid advancing twice or more times in the arterial tree, from the femoral artery with strings and catheters, threatening areas with possible complication risks due to the presence of atheromatosis in femoral, iliac arteries, abdominal, thoracic aorta, arch and ascending aorta.

The risk that complications may occur during these procedures has been described in the chapter of the Prior Art, emphasizing that one of the causes is, precisely, the advance of strings and catheters.

Consequently, it is stated that with the combined catheter of the present invention, the referred risk possibly decreases to the half, since it is only advanced once lifting simultaneously both catheters.

From the above mentioned constructive and functional principle, other additional benefits also arise, among which the following can be highlighted:
1. Only one entrance through the iliac and the aorta until the coronaries.
2. Less risk that a vascular accident (less than 50%), and any of the types above mentioned, are produced.
3. Less room time for each procedure. With the resulting use of the availability to performing more studies with better cost-benefit.
4. More speed to perform the study (50% quicker or half of the time or less).
5. Less exposure to rays of everyone involved (patient, technicians, nurses, medical personnel).
6. Having an additional resource, such as improving the JR curve and modifying it using the JL catheter.
7. Allowing the exchange of JL catheters (in case the initial curve does not allow the access to the left coronary) leaving the JR in the ascending aorta, and therefore, avoiding to cross one more time through the territory of the femorals, the descending aorta and arch, profiting by the resulting security of not moving or removing or damaging any type of atheroma or vessel.
8. To work with the same catheters, with known curves and with the same technique described by Judkins.

Risks:
1. To carelessly advance the JR catheter towards the left Coronary or the JL towards the right coronary.
2. To raise some impurities with the JR by advancing it first (without the JL outside) through femoral and iliac arteries.
3. That on tall patients or with arterial tortuosity, the catheter may not be long enough (due to its shortening, given by the constructive modification) and would prevent the possibility of achieving the selective catheterization of the Right Coronary artery. In this case, the solution is to withdraw it only a little (until the ascending aorta) and to advance a JR catheter of 1.35 mm via the inside (conventionally with the original size) and correctly achieve the objective.

To put into practice the mentioned use purposes and advantages, the combined catheter of the present patent application distinguishes itself due to the fact that in a preferred embodiment it comprises a JR catheter of a diameter of 2 mm of with an inferior length than the conventional ones, that, hand in hand with its distal end, defines a tapered and blunt section from where a JL catheter with a smaller diameter distally protrudes.

The invention highlights that the JR catheter includes, hand in hand with its proximal end, a haemostatic key in "Y".

The distal section of the JL catheter that protrudes from the distal end of the JR catheter includes its sections with a primary and secondary curve.

### Inventive Level

None of the angiography catheters currently known proposes, neither suggests, the constructive solution that arises from what has been stated in the previous paragraphs, reason why it is a proposal that, besides being novel, possesses a clear inventive activity.

### BRIEF DESCRIPTION OF DRAWINGS

To formalize the slightly discussed advantages, to which the users and skilled persons may add many other, and to facilitate the comprehension of the constructive, constitutive and functional characteristics of the invented combined catheter, an example of a preferred embodiment is illustrated below, schematically and in a determined scale, in the attached sheets, with the express clarification that, precisely, for being an example, the shame shall not be construed as limited or exclusive of the scope of protection of the present patent application, but simply assisting an intention solely explanatory and illustrative of the basic conception in which the same is based.
Figure 1 represents, in a side view, three models of catheters for left coronary of "Judkins JL".
Figure 2 represents, in a side view, three models of catheters for right coronary of "Judkins JR".
Figure 3 is a side view that represents, schematically, the combined catheter of the present invention, such as it is constituted to be applied. Extended details A and B are included, which show respective distinctive details of the invented embodiment.
Figure 4 is a complete scheme of the Aorta Artery, including a schematic embodiment of how the complete catheter is observed advancing from the femoral artery, within a valved introductor, and positions in the origin of the left coronary artery.
Figure 5 is a complete scheme of the Aorta Artery where a schematic embodiment is included of how it is observed the complete catheter after performing the Left Coronary Angiography.
Figure 6 is a complete scheme of the Aorta Artery representing the selective catheterization of the valved catheter of 2 mm in the ostium of the Right Coronary Artery.

In all the figures, to the same number and reference letters correspond the same or equivalent constitutive parts or elements of the set, according to the example chosen for the present explanation of the invented combined catheter.

### DESCRIPTION OF A PREFERRED EMBODIMENT

As it can be appreciated in Figure 1, the "Judkins" catheters for left coronary are currently know among which the following are represented:
● Model a) JL 3.5;
● Model b) JL 4;
● Model c) JL 5

The difference among them lies in the angles presented by their primary and secondary curvatures.

Also, in Figure 2 "Judkins" catheters for right coronary are represented, such as the following:
● Model d) JR 4;
● Model e) JR 5
● Model f) JR 6

Also, the difference among them lies in the curvature angles presented by their respective distal ends sections.

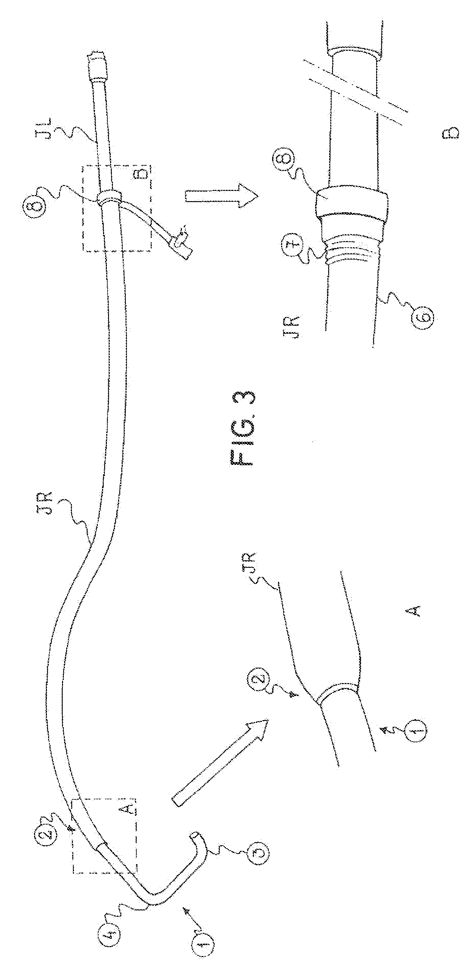

Now, observing Figure 3, it is possible to appreciate how the combined catheter of the present invention is constituted, which comprises a JR catheter of 2 mm with valved diameter, containing in its interior a respective JL catheter with a diameter of 1.35 mm, maintaining the constructive condition that the distal end (1) of the JL catheter is disposed projected outward from the distal end (2) of the JR catheter, with the special feature that said projected section includes the corresponding primary (3) and secondary (4) curvatures of the same JL catheter with a smaller diameter.

Also, if we appreciate the mentioned detail "A" included in this Figure 3, it is understood that the distal section (1) referred of the JL catheter is projected outward from a novel section of the distal end (2) belonging to the JR catheter JR, which distinguishes for presenting a specific constructive modification defining a tapered or blunt tip, which becomes apparent by a progressive and continuous decrease of its transversal section until practically reaching to the same diameter of the internal JL catheter (2) and, therefore, perfectly adapting to the external cylindrical surface of the referred JL catheter with a smaller diameter.

Likewise, the detail "B" of this Figure 3, shows that in correspondence with the proximal end of the JR catheter, an outstanding line (6) appears, which corresponds to a opaque radius mark incorporated to more clearly visualize said end by radioscopy, and therefore to control its advance.

The same detail "B" shows that this JR catheter is valved, for which it includes a threaded section (7) where the haemostatic valve (8) can be incorporated and withdrawn.

The presence of this valve (8), even though it increases the total value of the set, results important due to the fact that including a haemostatic valve or "haemostatic" key ensures a correct aspiration of blood and/or particles that may be accumulated in this catheter.

The basic function of the haemostatic valve (8) is to allow the performance of a washing or administration of saline or dextrose solution to prepare a catheter before introducing it and that the blood pressure does not reflow or exits; it is a valve that impedes the reflux.

In this sense, it is clarified that the conventional catheters do not include this kind of incorporated valve, which may also result useful to instill contrast or contrast material to dye the coronary without coming out (reflows).

The invention contemplates that said haemostatic valve (8) may be still and may function with a lateral connector with (3-way stop cock), such as if it were a valved introductor.

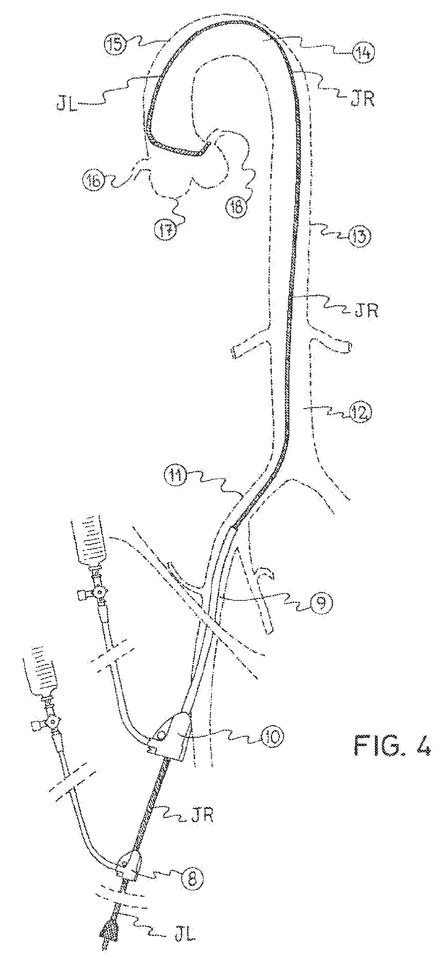

Figure 4 is the schematic representation of how the combined catheter of this invention is observed advancing from the femoral artery (9), within a valved introductor (10), and is positioned in the origin of the left coronary artery (11).

It is observed, in this figure, how the complete scheme of the Aorta Artery is composed from its origin and its different positions, among which the following are emphasized:
(11) = Iliac Artery
(12) = Abdominal Artery
(13) = Thoracic Artery
(14) = Aortic Arch
(15) = Ascending Aorta
(16) = Right Coronary
(17) = Origin of the Aorta Artery
(18) = Left Coronary

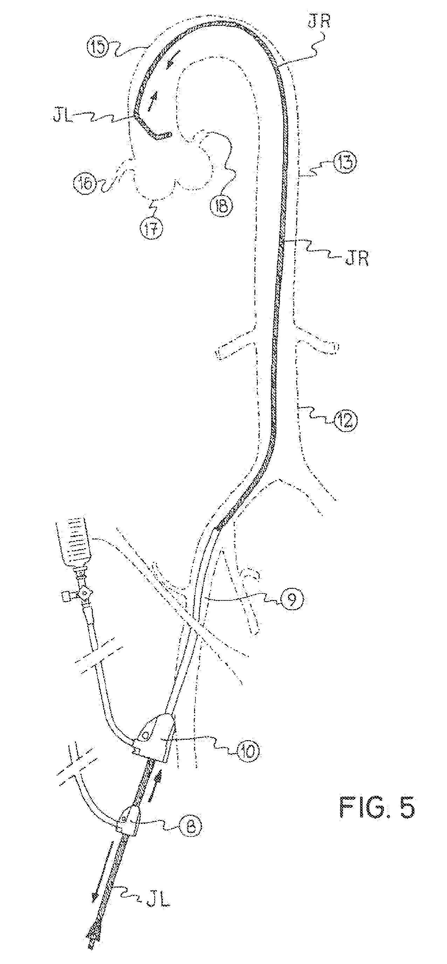

Observing now the Figure 5, it is appreciated how the procedure continues, after performing the Angiography of the left coronary.

In the first place, the JL catheter is withdrawn and then the JR catheter is advanced until the root of the Aorta Artery.

It is not necessary to completely withdraw the JL catheter, only up to the aortic arch (14) or even less, which allows keeping injecting contrast substance through the JL catheter, without affecting the right visualization of the right coronary.

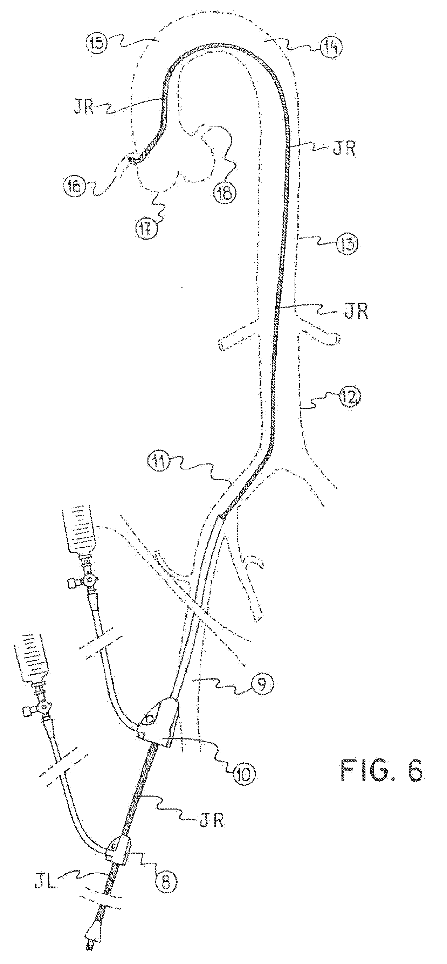

Once the valved JR catheter is positioned in the root of the Aorta, the common maneuvers to catheterize the ostium of the Right Coronary (16) are performed according to the Judkins technique (see Fig. 6).

In case the ostium has an ascending origin that makes difficult its selective catheterization (as seen in Fig. 6), it is possible to slowly advance the JL catheter 4 showing its end outside the JR 6 and trying to catheterize the Right Coronary (16) with the help of this more ascending curve.

If, with these maneuvers, the selective angiography of the Right Coronary is not feasible, the JL catheter is completely withdrawn and the JR catheter partially until the Thoracic Aorta (13), and then advancing with the latter.

Then, it is emphasized that the use of the combined catheter of the present invention allows the exchange of the JL and JR catheters (in case the initial curve does not allow the access to the left coronary), in which case the JR is left in the ascending aorta and therefore, avoiding to cross one more time through the territory of the femorals, the descending aorta and arch, profiting by the resulting security of not moving or removing or damaging any type of atheroma or vessel.

The mentioned fact of advancing within the valved JR catheter, developed with a different curve, results as an additional and wanted benefit to make the procedure faster and safer.

Bibliography:
1. Wingo JP, Nix ML, Greenfield LJ, Barnes RW: The blue toe syndrome: hemodynamics and therapeutic correlates of outcome. J Vasc Surg 1986; 3:475-480.
2. Kumpe DA, Zwerdlinger S, Griffin DJ: Blue digit syndrome: treatment with percutaneous transluminal angioplasty. Radiology 1988; 166: 37-44.
3. Crane C: Atherothrombotic embolisms to lower extremities in arteriosclerosis. Arch Surg 1967; 94:96-101.
4. Brenowitz JB, Edwards WS: The Management of atheromatous emboli to the lower extremities. Surg Gynecol Obstet 1976; 143:941-945.
5. Kempczinski RF: Lower-extremity arterial emboli from ulcerating atherosclerotic plaques. JAMA 1979; 241: 807-810.
6. Flory CM: Arterial occlusion produced by embolism from eroded atheromatous plaque. Am J Pathol 1945; 21: 549-565.
7. Hoye SJ, Teitelbaum S, Gore I, Warren R: Atheromatous embolization. N England J Med 1959; 261: 128-131.
8. Karmody AM, Powers SR, Monaco VJ, Leather RP: "Blue toe" syndrome: an indication for limb salvage surgery. Arch Surg 1976; 111:1263-1268.
9. Williams GM, Harrington D, Burdick J, White RI Jr: Mural thrombus of the aorta: an important, frequently neglected cause of larger Peripherals emboli. Ann Surg 1981; 194: 737-744.
10. Darsee JR: Cholesterol embolization: the great masquerader. South Med J 1979;72: 174-180.
11. Retan JW, Miller RE: Microembolic complications of atherosclerosis. Arch Intern Med 1966; 118: 534-545.
12. Harvey JC: Cholesterol cristal microembolization: a cause of the restless leg syndrome. South Med J 1976; 69: 269-272.
13. Banis JC, Rich N, Whelan TJ: Ischemia of the upper extremity due to noncardiac emboli. Am J Surg 1977; 134:131-139.
14. Lee BY, Brancata RF, Thoden WR, Madden JL: Blue digit syndrome: urgent indication for limb salvage surgery. Am J Surg 1984; 147:418-422.
15. Fisher DF, Clagett GP, Brigham RA, et al: Dilemmas in dealing with the blue toe syndrome: aortic versus Peripherals source. Am J Surg 1984; 148: 836-839.
16. Brewer ML, Kinnison ML, Peter BA, White RI Jr: Blue toe syndrome: treatment with anticoagulants and delayed percutaneous transluminal angioplasty. Radiology 1988; 166: 31.
17. Kwaan JHM, Connolly JE: Peripheral atheroembolism: an enigma. Arch Surg 1977;122:987-990.
18. Kwaan JH, Vander Molen R, Stremmer EA, Connolly JE: Peripheral embolism resulting from unsuspected atheromatous aortic plaques. Surgery 1975;78:583.
19. Roon AJ, Sauvage LR: Blue toe syndrome: a warning sign of unsuspected vascular injury. Surgery 1983; 93: 722-724.
20. Mehigan JT, Stoney RJ: Arterial microemboli and fibromuscular dysplasia of the external iliac arteries. Surgery 1977; 81: 484-486.
21. Samuels PB, Katz DJ: Diagnosis and management of arterial mural thromboemboli. Am J Surg 1977; 134:209-213.
22. Schechter DC: Atheromatous embolization to lower limbs. NY State J Med 1979; 79: 1180-1186.
23. Sixma JJ: Role of blood vessels, platelet and coagulation interactions in haemostasis, in Bloom AL, Thomas DP (eds): Haemostasis and Thrombosis. New York, Churchill Livingstone, 1981, pp 252-267.

## Claims

1. A combined catheter for angiography, specially conceived to perform a coronary catheterization by the femoral route with a prior punction and placing of 2mm introductors, using the "Judkins" catheter curves but without the need of first advancing the JL catheter and then the JR wherein it comprises a "Judkins" JR right coronary catheter, which contains in its interior a "Judkins" JL left coronary catheter that distally protrudes, disposing the section, defining the primary and secondary curves projecting from the distal end of the JR catheter of the right coronary, which is tapered and blunt in its own distal end section,

2. A combined catheter for angiography according to claim 1, wherein the JR catheter has incorporated a haemostatic valve.

3. A combined catheter for angiography according to claim 2, wherein the incorporated haemostatic valve, which allows washing, is a haemostatic valve in "Y".

4. A combined catheter for angiography according to claim 2, wherein the incorporated haemostatic valve, which allows washing, is disposed in the proximal end of the JR catheter.

5. A combined catheter for angiography according to claim 1, wherein the right coronary JR catheter has a diameter of 2mm, which presents a tapered distal end that decreases until approximately 1.35 mm, withholding the left coronary JL catheter that has a diameter of approximately 1.35 mm.
